# EUROPEAN PATENT SPECIFICATION

(11) **EP 4 240 870 B1**
(45) Date of publication and mention of the grant of the patent: **31.12.2025**
(21) Application number: 21889965.6
(22) Date of filing: 03.11.2021
(51) Int. Cl.: C12Q 1/6806, C12Q 1/6869, C40B 50/06, C12N 15/10

(54) **SYSTEMS AND METHODS FOR MAKING SEQUENCING LIBRARIES**
SYSTEME UND VERFAHREN ZUR HERSTELLUNG VON SEQUENZIERUNGSBIBLIOTHEKEN
SYSTÈMES ET PROCÉDÉS DE FABRICATION DE BANQUES DE SÉQUENÇAGE

(30) Priority: 03.11.2020 US 202063109035 P
(43) Date of publication of application: 13.09.2023
(60) Divisional application: 25214633.7
(73) Proprietor: Illumina, Inc., San Diego, CA 92122 (US)
(72) Inventor: MELTZER, Robert, Belmont, MA 02478 (US)
(74) Representative: Graham Watt & Co
(86) International application number: PCT/US2021/057857
(87) International publication number: WO 2022/098726

(56) References cited:
- EP-A1- 3 461 832
- WO-A1-2012/116146
- WO-A1-2020/014303
- WO-A2-2019/222523
- WO-A2-2021/046462
- US-A1- 2003 143 599
- US-A1- 2019 352 714

## Description

### Technical field

This invention relates to systems and methods for making sequencing libraries.

### Background

There is a growing attention toward personalized medicine. This is led by a fundamental shift from a one size fits all paradigm for patient treatment to one that embraces tailored therapies. Among the technologies driving the paradigm shift is RNA-seq. Using RNA-seq, medical personnel can quickly assess the health of individual patients to detect diseases and identify effective treatments. As such, RNA-seq provides clinically meaningful data for tailored therapies. However, the data is only meaningful to the extent that it is accurate.

Methods of RNA-seq involve a number of steps to promote accuracy. One step involves removing PCR duplicates (i.e., reads arising from PCR amplification of the same molecule). This step is important for RNA-seq analyses because biases that are inherent in PCR lead to some molecules being overrepresented. To address this, methods generally involve removing PCR duplicates before quantification. One approach to detecting PCR duplicates is with unique molecular identifiers (UMIs). UMIs have random sequences to uniquely label every molecule in a library before PCR. After sequencing, any reads with identical UMIs are identified as duplicates and removed.

Unfortunately, PCR errors can cause UMI sequences to change during PCR amplification and/or sequencing, thereby inhibiting their effectiveness as unique identifiers. Such PCR errors arise from nucleotide substitutions, nucleotide miscalling, insertions, and deletions. And because they change the UMI sequences, they can cause PCR duplicates to go undetected, thereby leading to the over quantification of some sequence reads and thus reducing the accuracy of RNA-seq analyses. WO 2020/014303 A1 relates to transposome enabled DNA/RNA-sequencing.

### Summary

The invention is defined by the attached claims. This invention relates to systems and methods for making libraries of molecularly distinct polynucleotides without unique molecular identifiers (UMIs). Instead, methods of the invention use a combination of random fragmentation and short random sequences to uniquely label polynucleotides. In particular, methods involve randomly fragmenting nucleic acids, preferably RNA, to create diversity within populations of identical molecules by producing fragments with ends at different random cleavage locations. Preferably, methods further include reverse transcribing the fragments in the presence of oligos with short random sequences (i.e., random N-mers), thereby creating polynucleotides with random N-mers copied therein. The result is a library of polynucleotides with distinct molecular identities based on unique combinations of the random cleavage locations and random N-mers. Accordingly, methods of the invention create libraries of polynucleotides that have multiple sources of distinct molecular identity. And as such, the identities of the polynucleotides are resistant to PCR errors and thus allow PCR duplicates to be accurately detected. Because PCR duplicates are accurately detected, assessments of RNA expression can be performed to provide reliable and clinically meaningful data from which personalized treatments can be made.

Moreover, methods of the invention are useful to create accurate and comprehensive sequencing libraries from single cells. Preferably, the single cell libraries are created in an emulsion format to isolate and individually profile separate cells inside droplets without the need for expensive microfluidic devices. The emulsions may be created with particles that template the formation of droplets inside a tube and segregate single cells therein. Accordingly, each droplet may provide an isolated reaction chamber, thereby allowing libraries of a multitude of single cells to be made from their corresponding reaction chambers in parallel. Accordingly, methods of the invention can provide a massively parallel, analytical workflow for preparing single-cell sequencing libraries.

In one aspect, the invention provides a method for preparing a sequencing library. The method includes obtaining a sample comprising RNA. The method further involves fragmenting the RNA to produce fragments with cleaved ends at random cleavage locations. The result is a diverse pool of fragments in which some previously identical molecules are made distinguishable on account of different cleavage locations. The fragments are reverse transcribed to make complementary DNA. Reverse transcription is preferably performed in the presence of oligos with short random N-mers (i.e., molecular diversity enhancers). The random N-mers may be random 4-mers or random 5-mers. The oligos are added to the cleaved ends of the fragments and copied into the cDNA during reverse transcription, thereby preserving identifying information from multiple sources of molecular diversity into the polynucleotides. Because the identifying information comes from multiple sources, e.g., sequences of the RNA fragments, the random cleavage locations and the random N-mers, the unique identities of the polynucleotides are resistant to PCR errors, thereby allowing PCR duplicates to be detected with impeccable accuracy.

Preferably, fragmenting is accomplished metal ion catalysis of RNA. Metal ion catalysis involves exposing the RNA to multivalent cations, for example, metal cations, such as Mg2+, Mn2+, or Zn2+, at high temperatures (e g., ~95 degrees Celsius). Exposure of the RNA to metal cations at high temperatures results in the hydrolysis of phosphodiester bonds, thereby cleaving the RNA at random positions along the RNA backbone. Advantageously, the size of fragments generated by this approach can be manipulated by adjusting the amount of time that the RNA is exposed to the high temperatures. For example, longer exposure times may lead to greater numbers of smaller fragments. As such, methods of the invention are useful for creating sequencing libraries with fragment sizes that are amenable to any of a variety of different sequencing platforms that require specific fragment sizes.

In preferred embodiments, the unique molecular identities of the polynucleotides are created from combinations of random cleavage locations and oligos. The oligos preferably include random N-mers. Random N-mers may provide 4^N different sequence combinations. The total number of the sequence combinations provided by the N-mers may be substantially less than an amount of distinct species of RNA present in the sample. As such, at least two of the polynucleotides, and preferably more, will comprise identical oligos. The relatively short size of the oligos provides multiple advantages over other methods for creating unique labels, such as UMIs. For example, because the oligos are short, they are less expensive to synthesize, less likely to suffer from PCR mutations, and reduce sequencing costs by requiring less sequencing.

Methods of the invention can make sequencing libraries from very low quantities of RNA (e.g., 5 nanograms or 10 nanograms). As such, methods of the invention are ideal for making sequencing libraries for single cell analyses. In particular, methods may include making a library from a sample comprising a mixture with cells. For example, the mixture may include thousands, tens of thousands, hundreds of thousands, millions, or at least about 10 million cells. Methods of the invention may be used to capture and partition any number of cells for making single cell libraries. For example, in some embodiments, the mixture includes two immiscible fluids, such as an aqueous solution and oil. Methods then include portioning the mixture into droplets that each include one or zero cells and lysing the cells inside the droplets to create libraries from of single cells in bulk. Accordingly, methods of the invention may include preparing libraries of single cells in multiple parallel reactions.

In some instances, methods of single cell library preparation further include particles that template the formation of the droplets upon partitioning the mixture. The particles may be gels with compartments that include reagents contained therein. For example, the particles may include reagents for any one of cell lysis, RNA fragmentation, or reverse transcription. The methods may include heating the mixture to a temperature that promotes cell lysis and hydrolysis of RNA in the presence of cations. Accordingly, fragmenting may occur within the droplets coincident with cell lysis, thereby allowing two library preparation steps to occur during an incubation reaction period.

In preferred embodiments, the RNA comprises mRNA and the template particles are linked to capture oligos comprising a poly-T sequence for capturing poly-A tails of the mRNA. Because poly-T sequences are complementary to 3' poly-A tails present on some mRNA fragments, the 3' poly-A tails of fragmented mRNA will hybridize with the poly-T sequences. Thus, methods may include the capture of sequences specific to mRNA, thereby allowing gene expression analysis from only the 3' ends of mRNA, which can significantly reduce sequencing costs by only sequencing the material that is of interest.

After hybridization of the poly-A tails with the poly-T sequences, the fragments may be reverse transcribed into complementary DNA in the presence of template switching oligos with random N-mers. Preferably, reverse transcription involves reverse transcriptase enzymes designed to add several nucleotides complementary to the template switching oligos at the ends of cDNA upon reaching the cleaved ends of the fragments. The template switching oligos attach to the nucleotides added by the enzymes and provide additional template for the enzymes to copy into cDNA, thereby creating the polynucleotides comprising the random N-mers and random cleavage locations.

Methods of the invention are useful to make libraries of polynucleotides that are uniquely identifiable. Accordingly, methods of the invention are useful for sequenced-based analyses (e.g., RNA-seq) which involve mapping sequence reads to a reference and counting unique reads (i.e., reads arising from distinct molecules). As such, methods of the invention may include amplifying polynucleotides to make amplicons and sequencing the amplicons to create a plurality of sequence reads. The sequence reads may be analyzed to identify and remove PCR duplicates with high accuracy. Identifying PCR duplicates preferably includes aligning the sequence reads to a human reference genome and identifying genomic coordinates that correspond with the random cleavage locations from the fragments. The positions that correspond with the random cleavage locations may be revealed by aligning the sequence reads to the reference genome and identifying ends of the aligned reads that are opposite of the ends corresponding with the poly-A tails. These positions may be referred to as transcript start positions. The positions corresponding with the poly-A tails may be referred to as transcript end positions. Because the RNA was fragmented before amplification, the likelihood that the transcript start positions of any two aligned sequence reads will be identical is very low.

Accordingly, sequences with the same transcript start positions may be identified as putative duplicates.

Methods may further include determining whether an identified putative duplicate is a true duplicate, or the unlikely result of two identical molecules being fragmented in the same location. To determine whether a sequence read is a true duplicate, sequences corresponding with the random N-mers may be compared. Any sequence reads having identical transcripts start positions and identical random N-mers with another sequence read may be identified as a true PCR duplicate.

In other aspects, the invention uses a direct tagmentation approach to make uniquely identifiable polynucleotides. Methods include preparing, in a tube, an aqueous mixture that includes nucleic acids, e.g., RNA. An oil is added to the tube, and the method includes shaking or vortexing the tube to partition the mixture into droplets surrounded by the oil. The nucleic acids may initially be in cells and the shaking step may cause droplets to form that contain the cells. The method may include lysing the cells within the droplets to release the nucleic acid into the droplets. In preferred embodiments, the nucleic acid includes mRNA.

Preferably the aqueous mixture includes a plurality of template particles, and shaking the sample vessel causes each template particle to serve as a template in the formation of one of the droplets. In certain embodiments, the template particles are linked to capture oligos, which are linked to the template particles at their 5' ends, and in which 3' ends of the capture oligos include a poly-T sequence. Each of the template particles may contain some of the reverse transcriptase enzymes. mRNA generally includes poly-A tails at 3' ends. The poly-A tails at the 3' ends of mRNA will attach to the 3' ends of the capture oligos by complementary base pairing. The mRNA may be reverse transcribed into complementary DNA (cDNA), thereby creating RNA/DNA hybrids.

Methods further include treating the RNA/DNA hybrids with enzymes that bind with RNA/DNA hybrids at random locations and integrate exogenous sequences at the randomly bound locations. For example, the enzymes may include bacterial transposases, such as, Tn5. The Tn5 enzymes may be associated with adapters that include random N-mers and optionally primers for PCR. The Tn5 may bind and cleave the RNA/DNA hybrids at random positions and ligate the adapters at the cleavage sites (e.g., 5' end of the cDNA). The RNA may be removed and the cDNA with the ligated adapters may be transcribed into polynucleotides. Each of the polynucleotides is uniquely identifiable based on combinations of random cleavage locations created by the Tn5 enzymes and random N-mer sequences.

### Brief description of the drawings

FIG. 1 shows a block diagram of a method for preparing a sequencing library.
FIG. 2 illustrates a sample prep tube comprising droplets.
FIG. 3 illustrates a sample prep tube following lysis of cells inside droplets.
FIG. 4 shows relative size distribution of mRNA fragments.
FIG. 5 diagrams a method for single-cell RNA-seq.
FIG. 6 shows a template particle linked to a capture oligo useful for initiating reverse transcription.
FIG. 7 illustrates the incorporation of a template switch oligo.
FIG. 8 illustrates the addition and extension of a sequencing adapter.
FIG. 9 illustrates the formation of a final library product.
FIG. 10 shows a workflow of library preparation by direct tagmentation.
FIG. 11 shows a workflow for directional tagmentation.
FIG. 12 shows a template particle linked to a capture oligo.
FIG. 13 shows a transposase bound with an RNA/DNA hybrid.
FIG. 14 illustrates the addition and extension of a sequencing adapter.
FIG. 15 illustrates the formation of a final library product.

### Detailed description

The invention is defined by the attached claims. High-throughput sequencing technologies yield vast numbers of short sequences (reads) from a pool of nucleic acid fragments. Over the last ten years, a wide variety of sequencing applications have been developed that estimate the abundance of a particular fragment by the number of reads obtained in a sequencing experiment (read counting) and then compare these abundances across biological conditions. Perhaps the most widely used read counting approach is RNA-seq, which seeks to compare the number of copies of each transcript in different cell types or conditions. Prior to sequencing, a PCR amplification step is normally performed to ensure sufficient DNA for sequencing and/or enrichment for fragments with successful adapter ligation. Biases in the PCR amplification step lead to particular sequences becoming overrepresented in the final library. In order to prevent this bias propagating to the quantification estimates, it is common to remove reads or read pairs with the same alignment coordinates, because they are assumed to arise through PCR amplification of the same molecule. This is appropriate where sequencing depth is low and thus the probability of two independent fragments having the same genomic coordinates are low, as with paired-end whole-genome DNA-seq from a large genome. However, the probability of generating independent fragments mapping to the same genomic coordinates increases as the distribution of the alignment coordinates deviates from a random sampling across the genome and/or the sequencing depth increases. For example, in RNA-seq, highly expressed transcripts are more likely to generate multiple fragments with the same genomic coordinates. The problem of PCR duplicates is more acute when greater numbers of PCR cycles are required to increase the library concentration, as in single-cell RNA-seq, or when the alignment coordinates are limited to a few distinct loci, as in individual-nucleotide resolution Cross-Linking and Immunoprecipitation (iCLIP). Random barcodes known as unique molecular identifiers (UMIs) were initially proposed as a method to count the number of mRNA molecules in a sample and have since been used to explicitly label PCR duplicates. By incorporating a UMI into the same location in each fragment during library preparation, but prior to PCR amplification, it is possible to identify PCR duplicates because they have both identical alignment coordinates and identical UMI sequences. Unfortunately, errors introduced during PCR amplification and sequencing can cause UMI sequences to change, resulting in artificial UMI sequences.

This invention relates to systems and methods for making libraries, such as sequencing libraries, of polynucleotides without the use of unique molecular identifiers (UMIs). In particular, the invention uses combinations of randomly integrated fragment sites and random N-mers, referred to as molecular diversity enhancers (MDEs), to make unique labels for polynucleotides. Methods of the invention create libraries of uniquely identifiable polynucleotides by randomly fragmenting nucleic acids, such as RNA, to generate a diverse pool of fragments with cleaved ends at random fragment locations (i.e., random cleavage locations). Fragments with cleaved ends in different fragment locations can be distinguishable even if they are from identical molecules. Preferably, methods also include reverse transcribing the fragments of RNA in the presence of molecular diversity enhancers (i.e., short random N-mers), thereby creating polynucleotides with the molecular diversity enhancers copied therein. The result is a library of polynucleotides that are uniquely identifiable based on combinations of the random cleavage locations and molecular diversity enhancers. Moreover, and in contrast with using UMIs, any PCR errors introduced into the MDE during amplification or sequencing are unlikely to impact the identification of PCR duplicates because the unique identity of the fragment is provided by at least two independent molecular sources, i.e., the random cleavage locations of the fragments and the MDEs. Accordingly, methods of the invention exploit multiple sources of diversity to ensure every polynucleotide is uniquely identifiable. And as such, methods of the invention may provide accurate RNA-seq analyses, which can be used to accurately diagnose and treat patients using personalized therapeutic approaches.

In addition, methods described herein provide sequencing libraries that are cheaper and faster to make. For example, methods disclosed herein provide a workflow that can be carried out in a single tube, thereby reducing costs of losing valuable material. These methods may also eliminate costly and labor-intensive steps by obviating the need for certain steps such as rRNA depletion, mRNA enrichment, double strand synthesis, DNA fragmentation, and A-tailing/adaptor ligation. Accordingly, methods of the invention offer cost-effective and time-efficient methods for RNA-seq libraries.

In preferred embodiments, methods of the invention are used to create single-cell sequencing libraries. As described herein, the libraries may be created with emulsions and template particles that segregate individual cells into droplets upon vortexing. The cells may be lysed inside the droplets, to release RNA. The RNA may be copied into uniquely labeled polynucleotides while still inside of the droplets, thereby allowing assessments of RNA from a multitude of single cells in one tube. Accordingly, methods of the invention provide a massively parallel, analytical workflow for preparing single-cell sequencing libraries. The methods are inexpensive, scalable, and accurate, while eliminating many of the drawbacks associated with convention RNA-seq.

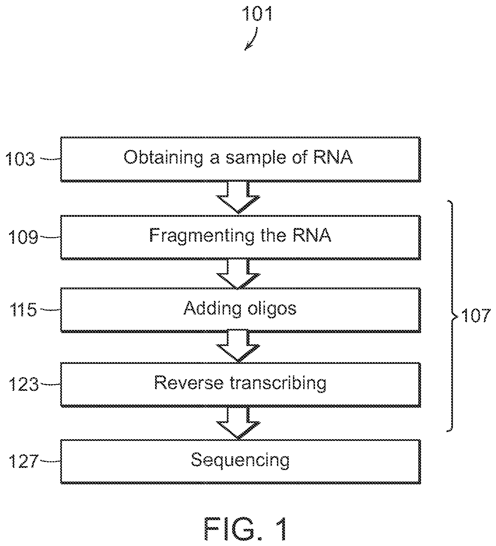

FIG. 1 shows a block diagram of a method 101 for preparing a sequencing library. The method 101 includes obtaining 103 a sample of RNA. Suitable samples may include whole or parts of blood, plasma, cerebrospinal fluid, saliva, tissue aspirate, microbial culture, uncultured microorganisms, swabs, or any other suitable sample. For example, in some embodiments, a blood sample is obtained 103 (e.g., by phlebotomy) in a clinical setting. Whole blood may be used, or the blood may be spun down to isolate a component of interest from the blood, such as peripheral blood monocytes (PBMCs).

After obtaining 103 the RNA, the RNA is fragmented 109. Fragmenting 109 may be carried out by any one of a number of different methods known in the art. For example, fragmenting 109 may be performed by physical methods, such as acoustic shearing and sonication, or by enzymatic methods, such as with a transposase, e.g., a Tn5 transposase. In preferred embodiments, however, fragmenting 109 is accomplished by exposing the RNA to high temperatures, e.g., about 95 degrees Celsius, in the presence of multivalent cations, such as, metal ions, for example, Mg2+, Mn2+, or Zn2+. For example, the RNA may be incubated in a solution comprising MgC12, at 95 degrees Celsius, for approximately five minutes. Importantly, the longer the exposure, the shorter the fragments. In some embodiments, it may be desirable to increase or reduce the exposure time so as to create fragments with sizes that are appropriate for a desired sequencing instrument, such as an Illumina sequencer.

Fragmenting 109 the RNA with metal ions may be used to generate fragments of RNA with cleaved ends at random cleavage locations. The cleavage locations are the locations where the RNA is fragmented (e.g., cleaved or broken), which will occur at substantially random positions across the RNA during fragmenting 109, leaving behind cleaved ends (i.e., the ends generated by fragmentation). These random cleavage locations are created by the hydrolysis of phosphodiester bonds included in the RNA backbone. Hydrolysis occurs when a deprotonated 2' OH of a ribose, acting as a nucleophile, attacks a 3' phosphorus in the phosphodiester bond of the sugar-phosphate backbone of the RNA. The phosphorus then detaches from the oxygen connecting it to an adjacent sugar, resulting in ester cleavage of the RNA backbone. This mechanism is referred to as RNA cleavage. Because phosphodiester bonds are present between every base of the RNA backbone, and each base is similarly accessible for hydrolysis, fragmenting 109 the RNA by the exposure to metal ions results in substantially random fragmentation of RNA.

Random fragmentation of RNA is desirable because it increases diversity of RNA within a sample. It is an insight of the invention that the increased diversity can be used to establish, or contribute to, the unique molecular identity of nucleic acids of a library. For example, RNA from a single species of RNA, i.e., RNA transcribed from the same genomic loci, has low to zero diversity as all the RNA from a single species have substantially identical sequences. Because their sequences are substantially identical, sequencing reads produced by sequencing that single species of RNA offer no inherent molecular tag for identifying unique sequence reads from PCR duplicates. However, by randomly fragmenting the RNA, that single species of RNA is made diverse on account of the fragments having random cleavage locations. In preferred embodiments, the diversity of the library is enhanced with the addition of molecular diversity enhancers (MDEs), which are short, random oligos at cleaved ends of fragmented RNA. Oligos, are strings of contiguous nucleotides of DNA or RNA or a mixture thereof. The length of the oligo is usually denoted by "-mer". For example, an oligo of six nucleotides is a hexamer, or 6-mer, while one of 25 nucleotides may be referred to as a 25-mer. The oligos preferably comprise a random sequence. The random sequence may be referred to as random N-mers. The random N-mer may make of the whole oligo, or just a portion thereof. The random N-mers may be, for example, random 6-mers, random 5-mers, random 4-mers, random 3-mers, etc. The random N-mer may comprise a random sequence or a not so random sequence. For example, the random N-mer may provide 4^N random different sequence combinations. For example, in some embodiments, the random N-mer is a 4-mer, and as such, provides 256 (i.e., 4^4) possible sequence combinations.

In most instances, on account of the relatively short size of the random N-mer, the total number of the sequence combinations provided by the N-mers is substantially less than an amount of distinct species of RNA present in the sample. As such, at least two of the polynucleotides, and preferably many more, will comprise identical oligos. This does not prevent uniquely identifying the polynucleotides, however, because the polynucleotides are also distinguishable by their random cleavage locations. Moreover, the relatively short sizes of the oligos provide multiple advantages over the use of other much longer barcodes, such as UMIs. For example, because the oligos are short, they are less expensive to synthesize, less likely to suffer from PCR mutations, and require less sequencing.

As discussed further in detail below, in preferred embodiments, the oligos comprise template switching oligos. The oligos may further comprise random N-mers. Template switch oligos are oligos that hybridize to untemplated C nucleotides added by the reverse transcriptase during reverse transcription. The template switching oligos often add common sequence to full length cDNA, which may be used for downstream cDNA amplification. Preferably, the template switching oligos comprise the random N-mers. Preferably the random N-mers are one of 5-mers or 4-mers, or a combination thereof.

Methods 101 of the invention further include reverse transcribing 123 or copying the fragments and oligos into polynucleotides with unique labels. Reverse transcribing 123 may be performed with a reverse transcriptase, such as, for example, the reverse transcriptase sold under the trade name SMARTScribe by Takara Bio. This enzyme, in the presence of oligos, such as template switch oligos, not only reverse transcribes the RNA, but also performs template switching functions. Accordingly, when the reverse transcriptase reaches the cleaved ends (i.e., the end of the RNA fragments), it adds three to four additional "C" bases. Then, the template switching oligos, which include three RNA bases (rGrGrG), bind to the "C", thereby adding 115 the oligos at (i.e., adjacent to) the cleaved ends. Afterwards, the reverse transcriptase proceeds to copy the template switch oligos into the cDNA, thereby creating polynucleotides comprising sequences from the fragments with the random cleavage locations, and the oligos with random N-mers.

The polynucleotides may be amplified after reverse transcription. The polynucleotides may also be barcoded with, for example, sequence platform specific primers (e.g., P5 and P7). Preferably, amplification is performed using polymerase chain reaction (PCR). The majority of PCR methods rely on thermal cycling. Thermal cycling exposes reactants to repeated cycles of heating and cooling to permit different temperature-dependent reactions - specifically, polynucleotide melting and enzyme-driven nucleic acid replication. PCR employs two main reagents: primers (which are short single strand fragments known as oligonucleotides that are a complementary sequence to the target DNA region) and a DNA polymerase. In the first step of PCR, the two strands of the DNA double helix are physically separated at a high temperature in a process called nucleic acid denaturation. In the second step, the temperature is lowered and the primers bind to the complementary sequences of the polynucleotides. The two strands then become templates for DNA polymerase to enzymatically assemble a new DNA strand from free nucleotides, the building amplicons (i.e., blocks of DNA). As PCR progresses, the DNA generated is itself used as a template for replication, setting in motion a chain reaction in which the original DNA template is exponentially amplified.

The oligonucleotide primers preferably include sequencing primers, such as P5 and P7 sequences, which are used for Illumina sequencing.

Amplifying the polynucleotides creates amplicons - pieces of DNA or RNA that are the product of amplification or replication events. The amplicons may be stored, for example, at -20 degrees Celsius, or may be analyzed. Analyzing amplicons preferably involves sequencing.

Sequencing 127 may be performed by any method known in the art. An example of a sequencing technology that can be used is Illumina sequencing. Illumina sequencing is based on the amplification of DNA on a solid surface using fold-back PCR and anchored primers.

Genomic DNA is fragmented and attached to the surface of flow cell channels. Four fluorophore-labeled, reversibly terminating nucleotides are used to perform sequential sequencing. After nucleotide incorporation, a laser is used to excite the fluorophores, and an image is captured, and the identity of the first base is recorded. Sequencing according to this technology is described in U.S. Pub. 2011/0009278, U.S. Pub. 2007/0114362, U.S. Pub. 2006/0024681, U.S. Pub. 2006/0292611, US 2011/0014657 A1, US 2008/0262747 A1, US 2004/0091903 A1, US 2005/0042673 A1, U.S. Pat. 6,306,597, WO98/13523, U.S. Pat. 6,828,100, US 2003/0013101 A1, and US 2002/0025529 A1,. In preferred embodiments, an Illumina Mi-Seq sequencer is used.

Sequencing 127 creates sequence reads, i.e., an inferred sequence of base pairs (or base pair probabilities) corresponding to all or part of a single nucleic acid. The sequence reads may be analyzed to determine expression of RNA associated with genes based on unique reads that correspond to those genes. Analyzing the sequence reads may be performed using known software and following multistep procedures that are known in the art. For example, first, the quality of each sequence read, i.e., FASTQ sequence, may be assessed using the software FASTQC. Next, the reads may be trimmed using, for example, using Trimmomatic software.
The trimmed sequence reads may then be mapped to a human genome using with, for example, HISAT2 software. HISAT2 output files in a SAM (sequence alignment/map format), which may be compressed to binary sequence alignment/map files. Other methods useful for processing and analyzing sequence reads are discussed in U.S. Pat. No. 8,209,130

Determining gene expression generally involves counting numbers of sequence reads that uniquely map to a human reference genome. Mapping may be performed with a computer program. The program may partition the reference genome into bins. The sequence reads may be aligned to the partitioned reference genome and the number of unique read sequences corresponding with bins may be counted and then normalized by LOESS fit.

Identifying unique sequence reads is critical for accurate gene expression analyses because of biases inherent in PCR. PCR biases result in the overamplification of some polynucleotides during amplification and therefore lead to the overrepresentation of some polynucleotides after sequencing. Accordingly, quantifying gene expression by counting every sequence read would result in over quantification of those polynucleotides that were overamplified. As such, the sequence reads should be analyzed to identify and remove or ignore PCR duplicates and only count the sequence reads that are products of distinct nucleic acid molecules.

This disclosure provides unique and accurate methods for identifying PCR duplicates. Methods may involve aligning the sequence reads to a reference genome and identifying coordinates of transcript start positions. Transcript start positions are the positions of the nucleotides that correspond with nucleotides present on the 5' ends of cleaved mRNA. In contrast, the transcript end position corresponds with the 3'end, i.e., the poly-A tail. Because the RNA was fragmented before amplification, the likelihood that transcript start positions of any two aligned sequence reads will be identical is very low.

Accordingly, methods for identifying PCR duplicates may involve aligning the sequence reads to the human reference genome and identifying the genomic coordinates of transcript start positions of the sequence reads. The genomic coordinates describe the precious position of the start position on a chromosome. Any two sequence reads that have identical transcript start positions are indicative of a putative duplicate, which may be identified or flagged. Putative duplicates may be removed from further analysis, or, in preferred embodiments, MDEs of the putative duplicates may be analyzed to determine whether the putative duplicates are true PCR duplicates. For example, the putative duplicates that have identical MDE sequences will be identified as true PCR duplicates and at least one of those PCR duplicates can be thrown out or ignored such that each unique sequence read is counted one time.

In preferred embodiments, some steps (107) of the method 101 are performed using single cells inside droplets. The method may be performed with a sample comprising a mixture with cells, and preferably template particles. The mixture preferably includes two immiscible fluids such as an aqueous fluid and oil. The mixture is sheared, e.g., vortexed, to generate an emulsion with template particles that serve to template the formation of droplets and segregate individual cells into the droplets. Because the cells are individually segregated into droplets, the cells may be individually profiled in parallel. This method provides a massively parallel, analytical workflow for analyzing single cells that is inexpensive, scalable, and accurate.

For example, methods of the invention may include combining template particles with cells in a first fluid and then adding a second fluid that is immiscible with the first fluid to the mixture. The first fluid is preferably an aqueous fluid. While any suitable order may be used, in some instances, a tube may be provided comprising the template particles. The tube can be any type of tube, such as a sample preparation tube sold under the trade name Eppendorf, or a blood collection tube, sold under the trade name Vacutainer. The sample may be a blood sample and may be added directly to the tube using a pipette.

The fluids can be sheared to generate a monodisperse emulsion with droplets. To generate a monodisperse emulsion, the presently disclosed method includes a step of shearing the mixture provided by combining cells and template particles in an aqueous fluid with the immiscible fluid. Any suitable method or technique may be utilized to apply a sufficient shear force to the second mixture. For example, the second mixture may be sheared by flowing the second mixture through a pipette tip. Other methods include, but are not limited to, shaking the second mixture with a homogenizer (e.g., vortexer), or shaking the second mixture with a bead beater. In some embodiments, vortex may be performed for example for 30 seconds, or in the range of 30 seconds to 5 minutes. The application of a sufficient shear force breaks the second mixture into monodisperse droplets that encapsulate one of a plurality of template particles. After vortexing, a plurality (e.g., thousands, tens of thousands, hundreds of thousands, one million, two million, ten million, or more) of aqueous partitions is formed essentially simultaneously. Vortexing causes the fluids to partition into a plurality of monodisperse droplets. A substantial portion of droplets will contain a single template particle and a single target cell. Droplets containing more than one or none of a template particle or target cell can be removed, destroyed, or otherwise ignored.

The next step of the method is to lyse the cells. Cell lysis may be induced by a stimulus, such as, for example, lytic reagents, detergents, or enzymes. Reagents to induce cell lysis may be provided by the template particles via internal compartments. Preferably, lysing involves heating the monodisperse droplets to a temperature sufficient to release lytic reagents contained inside the template particles into the monodisperse droplets. This accomplishes cell lysis of the target cells, thereby releasing nucleic acids, such as RNA, and preferably mRNA, inside of the droplets that contained the target cells.

Cell lysis may occur coincident with the fragmentation of the RNA. Fragmentation, as described above, is preferably performed with metal ions, which cause the random hydrolysis of RNA at high temperatures. After lysing target cells inside the droplets, mRNA is released. The mRNA may be used to create a sequencing library as described in FIG. 1.

Methods and systems of the invention may use template particles to template the formation of monodisperse droplets and isolate single target cells. The disclosed template particles and methods for targeted library preparation thereof leverage the particle-templated emulsification technology previously described in, Hatori et. al., Anal. Chem., 2018 (90):9813-9820. Essentially, micron-scale beads (such as hydrogels) or "template particles" are used to define an isolated fluid volume surrounded by an immiscible partitioning fluid and stabilized by temperature insensitive surfactants.

In practicing the methods as described herein, the composition and nature of the template particles may vary. For instance, in certain aspects, the template particles may be microgel particles that are micron-scale spheres of gel matrix. In some embodiments, the microgels are composed of a hydrophilic polymer that is soluble in water, including alginate or agarose. In other embodiments, the microgels are composed of a lipophilic microgel.

In some embodiments, the presently disclosed template particles may further comprise materials which provide the template particles with a positive surface charge, or an increased positive surface charge. Such materials may be without limitation poly-lysine or Polyethyleneimine, or combinations thereof. This may increase the chances of association between the template particle and, for example, a cell which generally has a mostly negatively charged membrane.

Other strategies may be used to increase the chances of template particle-target cell association, which include creation of specific template particle geometry. For example, in some embodiments, the template particles may have a general spherical shape but the shape may contain features such as flat surfaces, craters, grooves, protrusions, and other irregularities in the spherical shape.

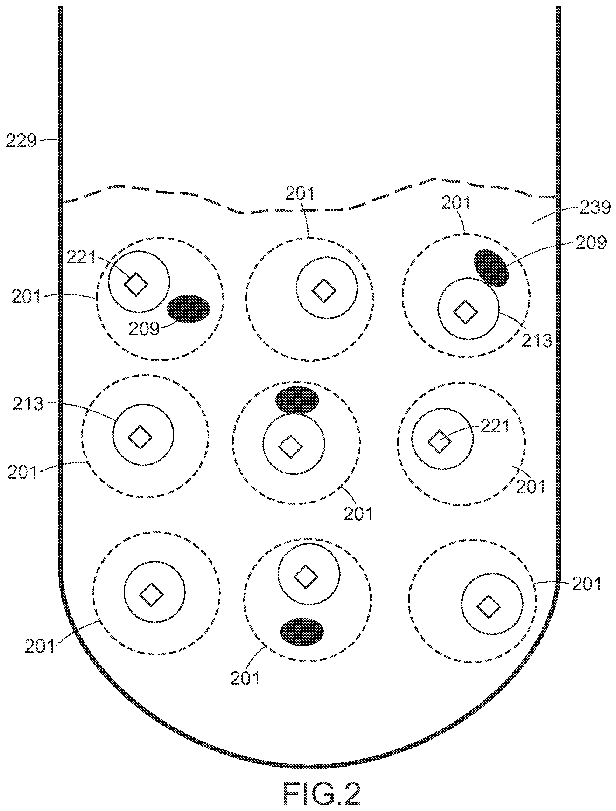

FIG. 2 illustrates a sample prep tube 229 comprising droplets 201. In particular, the sample prep tube 229 comprises a plurality of monodisperse droplets generated by shearing a mixture 239 according to preferred methods of the invention. Preferably, each of the droplets 201 comprise a template particle 213 and a single target cell 209. The template particles 213 may comprise crater-like depressions (not shown) to facilitate capture of single cells 209. The template particles 213 may further comprise an internal compartment 221 to deliver one or more reagents into the droplets 201 upon stimulus.

In some embodiments, the template particles contain multiple internal compartments. The internal compartments of the template particles may be used to encapsulate reagents that can be triggered to release a desired compound, e g., a substrate for an enzymatic reaction, or induce a certain result, e.g. lysis of an associated target cell. Reagents encapsulated in the template particles' compartment may be without limitation reagents selected from buffers, salts, lytic enzymes (e.g. proteinase k), other lytic reagents (e. g. Triton X-100, Tween-20, IGEPAL), nucleic acid synthesis reagents, or combinations thereof.

Lysis of single target cells occurs within the monodisperse droplets and may be induced by a stimulus such as heat, osmotic pressure, lytic reagents (e.g., DTT, beta-mercaptoethanol), detergents (e.g., SDS, Triton X-100, Tween-20), enzymes (e.g., proteinase K), or combinations thereof. In some embodiments, one or more of the said reagents (e.g., lytic reagents, detergents, enzymes) is compartmentalized within the template particle. In other embodiments, one or more of the said reagents is present in the mixture. In some other embodiments, one or more of the said reagents is added to the solution comprising the monodisperse droplets, as desired.

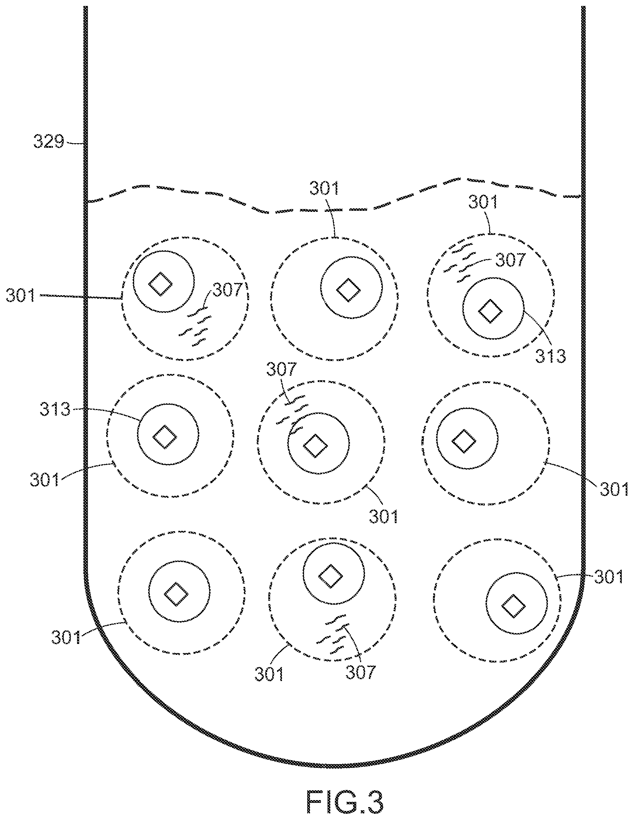

FIG. 3 illustrates a sample prep tube 329 following lysis of single cells inside droplets 301. The droplets 301 comprises template particle 313 and released mRNA 307. Preferably the mRNA 307 is fragmented. Fragmentation may occur substantially coincident with cell lysis. Preferably, fragmentation is achieved by incubating the sample at high temperatures and in the presence of metal ions.

In preferred embodiments, template particles comprise a plurality of capture probes. Generally, the capture probe of the present disclosure is an oligonucleotide. In some embodiments, the capture probes are attached to the template particle's material, e.g. hydrogel material, via covalent acrylic linkages. In some embodiments, the capture probes are acrydite-modified on their 5' end (linker region). Generally, acrydite-modified oligonucleotides can be incorporated, stoichiometrically, into hydrogels such as polyacrylamide, using standard free radical polymerization chemistry, where the double bond in the acrydite group reacts with other activated double bond containing compounds such as acrylamide. Specifically, copolymerization of the acrydite-modified capture probes with acrylamide including a crosslinker, e.g. N,N'-methylenebis, will result in a crosslinked gel material comprising covalently attached capture probes. In some other embodiments, the capture probes comprise Acrylate terminated hydrocarbon linker and combining the said capture probes with a template particle will cause their attachment to the template particle.

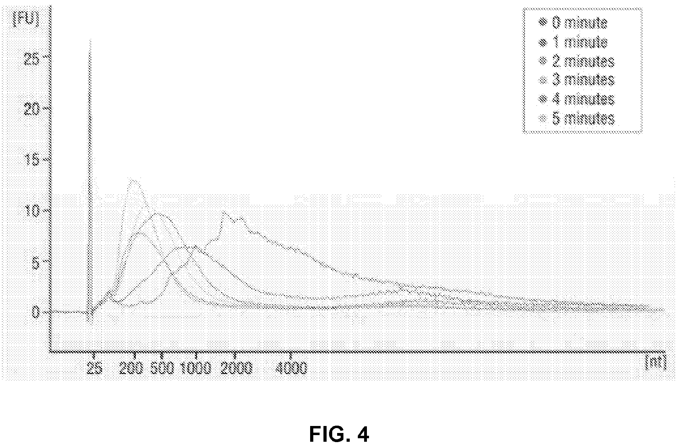

FIG. 4 shows relative size distribution of mRNA fragments. Size distribution data are produced by exposing mRNA to high temperatures and metals ions for the time periods indicated. The data show fragment sizes of mRNA are tunable by adjusting incubation times. The metal ions may include divalent cations such as Mg2+ or Mn2+, which promote RNA degradation in high-temperature conditions. This workflow is effectively implemented for controlled mRNA sizing. This may be used as a component in single cell RNA-seq, for example, as described in Duclos, 2019, Characterizing smoking-induced transcriptional heterogeneity in the human bronchial epithelium at single-cell resolution, Science advances, vol. 5,12. In some instances, the mRNA may be isolated from single cells in wells, or droplets, and converted to cDNA by reverse transcription, and then amplified as RNA through in-vitro transcription. The resulting libraries may then be fragmented by Mg2+ incubation and converted again to cDNA for library preparation according to some methods described herein.

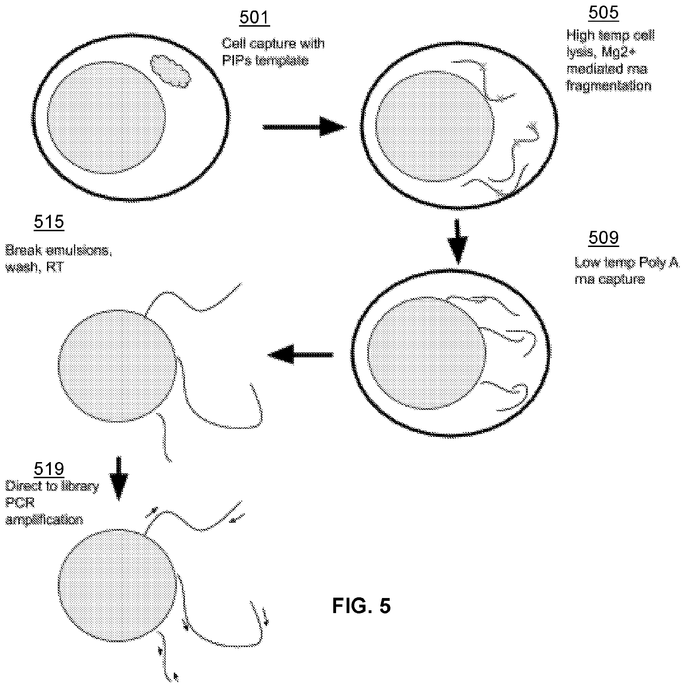

FIG. 5 diagrams a method for single-cell RNA-seq. After cell suspensions are introduced to template particles in a pre-equilibrated buffer, such as PP05 buffer with added Mg2+, droplets are generated by vortexing the mixture, as discussed above, to capture 501 single cells with individual template particles. The resulting emulsion is then rapidly cycled to a high (94 degrees Celsius) temperature 505, and then dropped back to 4 degrees Celsius 509 on a thermocycler. This process will induce cell lysis and controlled RNA degradation nearly simultaneously. At 4 degrees Celsius hybridization of only poly-A tailed 3' RNA fragments to poly-T decorated capture primers is promoted. Particle-bound capture primers in this application may comprise an acrydite linker, a PE1 priming sequence, a particle barcode, a random sequence, and a poly-T capture moiety.

After hybridization, emulsions may be broken 515 and the aqueous solution rapidly diluted with EDTA containing buffers in order to quench any further RNA degradation. Templates may be washed to exchange to an appropriate reverse-translation buffer, and reverse transcription may be performed with a modified template-switch oligonucleotide comprising an MDE. In some instances, the template-switching oligo comprises a P7 PCR adapter with terminating rGrGrG. After reverse transcription, the product may be directly amplified with a P5-PE1 hybrid oligo and P7 index primer directly into a sequencing library 519. The library may be sequenced to assess RNA expression, for example, as described in Hrdlickova, 2017, RNA-Seq methods for transcriptome analysis, Wiley Interdiscip Rev RNA 8(1): 10.1002.

This approach may depend, however, on direct controlled fragmentation of the genomic mRNA present in the sample. Presumably, every 3' fragment with a poly-A tail may be captured onto the template particles. The random fragmentation provided by divalent cation incubation, however, may make some of that population too short for further analysis. Other applications perform fragmentation of RNA or DNA after an initial round of amplification, as that may reduce the possibility of loss of unique initial molecules. This approach may also depend on the ability to induce controlled fragmentation and then stop further fragmentation by returning the sample to cold temperature until emulsions can be broken and the assay quenched by chelation (EDTA).

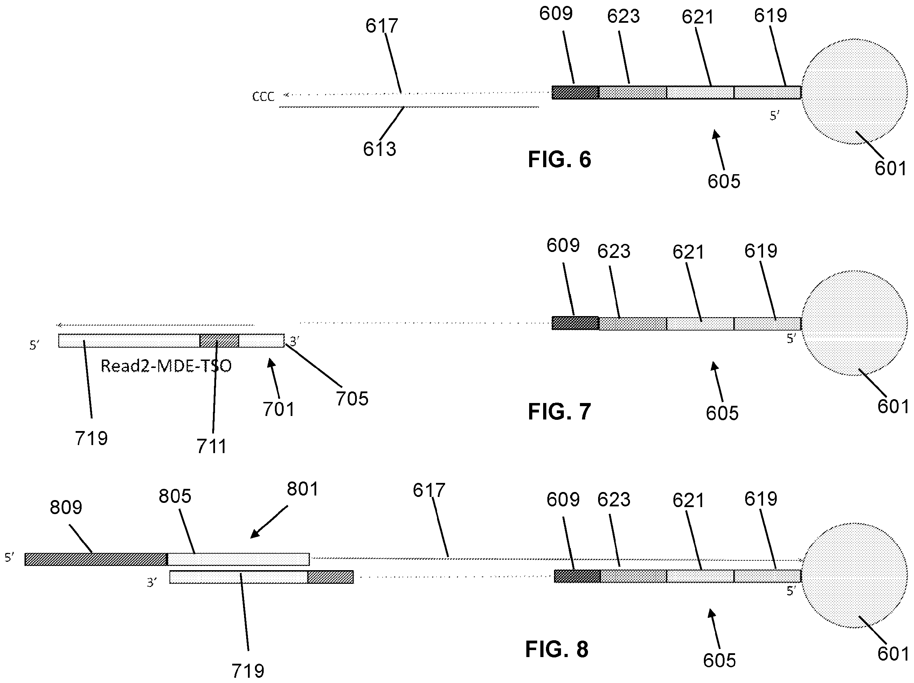

FIG. 6 shows a template particle 601 linked to a capture oligo 605 useful for initiating reverse transcription. As shown, the particle 601 is linked to (among other things) mRNA capture oligos 605 that include a 3' poly-T region 609 (although sequence-specific primers or random N-mers may be used). Where the sample includes cell-free RNA, the capture oligo hybridizes by Watson-Crick base-pairing to a target in the RNA and serves as a primer for reverse transcriptase, which makes a cDNA copy of the RNA. Where the initial sample includes intact cells, the same logic applies but the hybridizing and reverse transcription occurs once a cell releases RNA (e g., by being lysed).

In preferred embodiments, the target RNAs are mRNAs 613. For example, methods of the disclosure may be used to make a cDNA library useful for making an expression profile of a cell. Where the target RNAs are mRNAs, the particles 601 may include mRNA capture oligos 605 useful to at least synthesize a first cDNA copy 617 of an mRNA 613. The particles 601 may further include cDNA capture oligos with 3' portions that hybridize to cDNA copies of the mRNA. For the cDNA capture oligos, the 3' portions may include gene-specific sequences or hexamers. As shown, the mRNA capture oligos 605 include, from 5' to 3', a SMART site 619, a PE1 sequence 621, a cell or droplet barcode 623, and a poly-T segment 609.

As shown, the capture oligo 605 hybridizes to a target mRNA 613 by complementary base pairing with the 3' poly- A tail. A reverse transcriptase then binds and initiates synthesis of a cDNA molecule 617 from the mRNA 613. Note that the mRNA 613 is connected to the particle 601 non-covalently, by Watson-Crick base-pairing. The cDNA 617 that is synthesized may be covalently linked to the particle 617 by virtue of the phosphodiester bonds formed by the reverse transcriptase.

At the end of the mRNA 613, reverse transcriptase adds three to four additional nucleotides, for example, "C" bases. The additional bases are complementary to a template switch oligo, which attaches at a 5' end of the mRNA 613, providing more template for cDNA synthesis.

FIG. 7 shows the addition of a template switch oligo 701. The template switch oligo 701 is attached to the RNA bases (rGrGrG) 705 by complementary base pairing adjacent to the mRNA (not shown). The template switch oligo 701 preferably includes a random N-mer (i.e., a MDE), such as a random 5-mer, 711, and optionally, a primer (Read 2 primer) 719 for attaching a sequencing primer. Reverse transcriptase copies the template switch oligo 701 into the cDNA. At this stage, RNaseH may be introduced to degrade the mRNA 613.

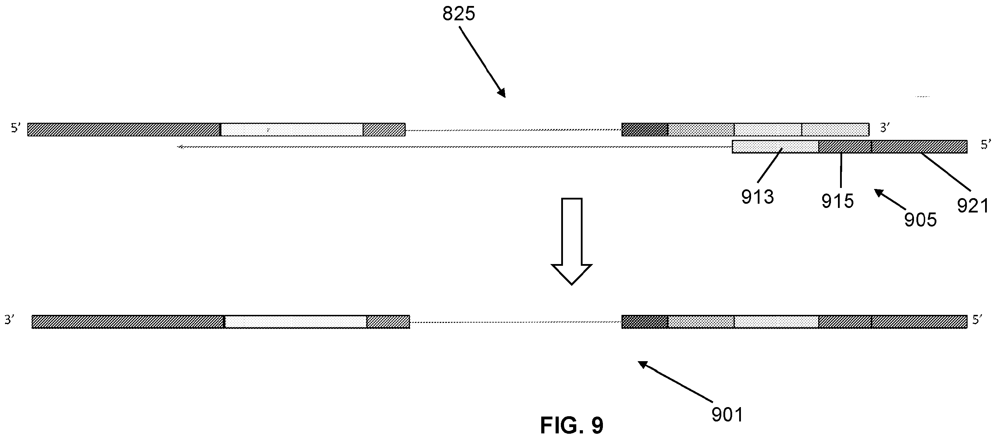

FIG. 8 illustrates the addition and extension of a sequencing adapter 801. The adapter 801 includes a first sequence 805 complementary to the primer 719 and a sequencing primer 809, such as P7. The adapter 801 will hybridize to, and prime the copying of, the cDNA 617, to create a polynucleotide 825 (Fig 9). Afterwards, the capture oligo 605 may be separated from the bead.

FIG. 9 illustrates the formation of a final library product 901. In this example, the final library product 901 is formed by the PCR amplification of the polynucleotide 825 using a P5-PE1 primer 905. The P5-PE1 primer includes a hybridization sequence 913 that is complementary to the PE1 site of the polynucleotide. The P5-PE1 primer may further include an index, such as an 15 index 915, and a P5 index 921.

As shown, the final library product 901 may include certain primer and index sequences, such as, P5s and P7s. Those sequences may be any arbitrary sequence useful in downstream analysis. For example, they may be additional universal primer binding sites or sequencing adaptors. For example, either or both of the P5s and P7s may be arbitrary universal priming sequence (universal meaning that the sequence information is not specific to the naturally occurring genomic sequence being studied, but is instead suited to being amplified using a pair of cognate universal primers, by design). The index segment may be any suitable barcode or index such as may be useful in downstream information processing. It is contemplated that the P5 sequences, the P7 sequence, and the index segment may be the sequences use in NGS indexed sequences such as performed on an NGS instrument sold under the trademark ILLUMINA, and as described in Bowman, 2013, Multiplexed Illumina sequencing libraries from picogram quantities of DNA, BMC Genomics 14:466 (esp. in Figure 2).

In other embodiments, methods of the disclosure may generate uniquely labeled sequencing libraries by a direct tagmentation approach. Direct tagmentation may be performed with transposases, such as, Tn5. For example, as discussed in Lin, 2020, RNA sequencing by direct tagmentation of RNA/DNA hybrids, PNAS117 (6) 2886-2893. In brief, the Tn5 transposase randomly binds and cuts double-stranded RNA/DNA to directly fragment RNA/DNA hybrids generated by reverse transcription and tagment the hybrids with a random N-mer.

Accordingly, some embodiments of the invention use Tn5 transposase to directly tagment RNA/DNA hybrids and form polynucleotide libraries with MDEs (i.e., oligos comprising random N-mers. In particular, Tn5, a RNase H superfamily member, binds to RNA/DNA hybrids similarly as to dsDNA and effectively fragments and then ligates a desired oligo onto the hybrid. The desired oligo is preferably the MDE. This method may be used to improve the robustness of low-input RNA-seq with a simplified experimental procedure. The method works with various amounts of input sample, from single cells to bulk RNA, with a dynamic range spanning six orders of magnitude. The method may provide superior cross-sample robustness and comparable detectability for both bulk RNA and single cells compared with other conventional methods and may provide a unique solution for small bulk samples that existing approaches struggle to handle. Furthermore, this easy-to-operate protocol is scalable and cost-effective, holding promise for use in high-quality and high-throughput RNA-seq applications.

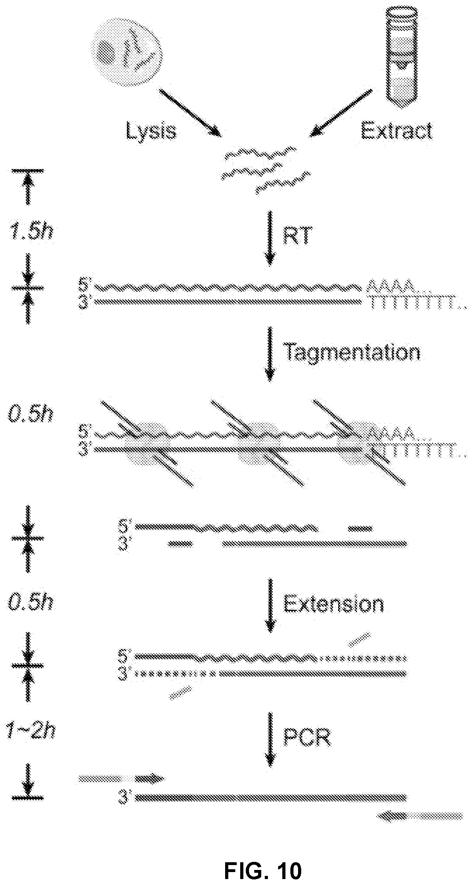

FIG. 10 shows a workflow of library preparation by direct tagmentation. The input can be RNA from lysed single cells, preferably in droplets, or extracted bulk RNA. The RNA is reverse transcribed with reverse transcriptase according to known methods to create cDNA/RNA duplexes. After reverse transcription, the cDNA/RNA hybrids are directly tagmented by Tn5 enzymes bound with adapters to add those adapters to the ends to the cDNA/RNA hybrids. Preferably, the adapters include at least one random N-mer (i.e., an MDE) and primers for amplification of the final library. After tagmentation, the tagged cDNA/RNA duplexes may be subjected to gap-repair and enrichment PCR.

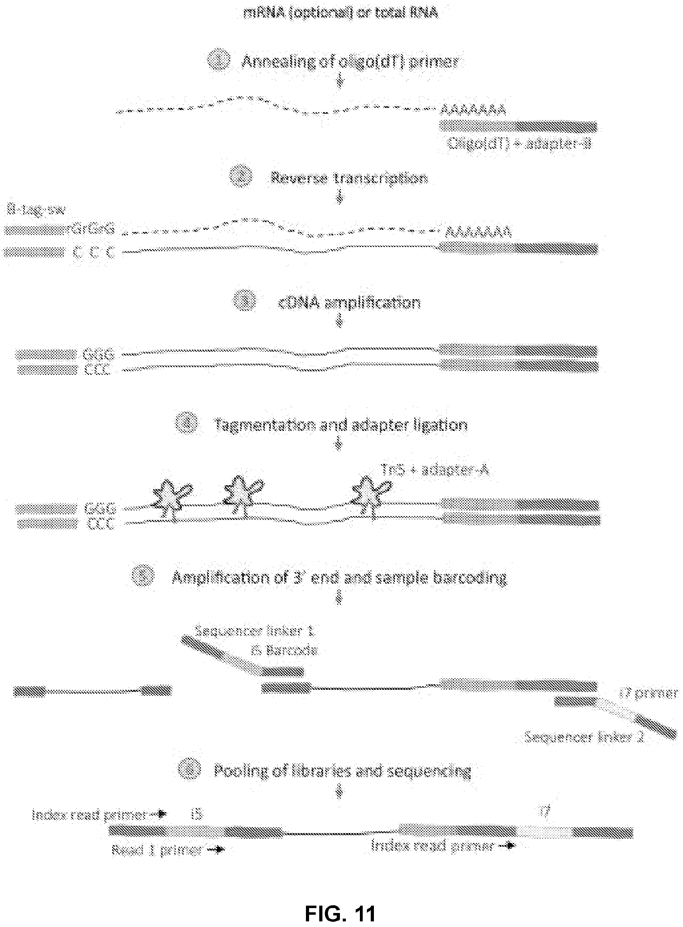

FIG. 11 shows a workflow for directional tagmentation. This tagmentation approach is useful for 3' end capture and analysis of mRNAs. The steps of the method are shown. In brief, mRNA or total RNA from lysed cells are mixed with an oligo and incubated at 65° for 3 min. The oligo may include specific primers for amplifying final libraries, such as an adapter-B sequence complementary to an i7 primer. The oligo may further include a poly-T sequence of, for example, 30 nucleotides that hybridizes with poly-A tails of mRNA. Importantly, the use of this oligo to prime a first strand cDNA synthesis may result in libraries enriched for the 3' end of mRNA.

Reverse transcription can be performed using a reverse transcriptase such as the reverse transcriptase sold under the trade name SMARTScribe and in the presence of a template switching oligo. The template switching oligo may include an MDE. The template switching oligo allows for template switching at the 5' end of the mRNA molecule to incorporate the oligo with the MDE and optionally a universal 3' sequence during first strand cDNA synthesis. The template switching oligo may include features designed to prevent concatemerization of the oligo, a common problem when the input RNA amount is low. Synthesis of the first cDNA strand may be performed using a thermocycler at 42 degrees Celsius for 1h, followed by 15 minutes at 70 degrees Celsius to inactivate the reverse transcriptase. Afterwards, the cDNA may be amplified. The cDNA may be amplified by PCR using commercially available kits such as the kit sold under the trade name OneTaq HS by New England Biolabs. After amplification, the RNA/DNA duplexes may be subjected to tagmentation and adapter ligation.

During tagmentation and adapter ligation, Tn5 bound adapter (adapter-A) complexes bind with the double RNA/DNA duplexes. The duplexes are cut by the enzymatic activity of the Tn5 complexes and the adapters are ligated. Afterwards, the products of the tagmentation reaction may be amplified using the adapters.

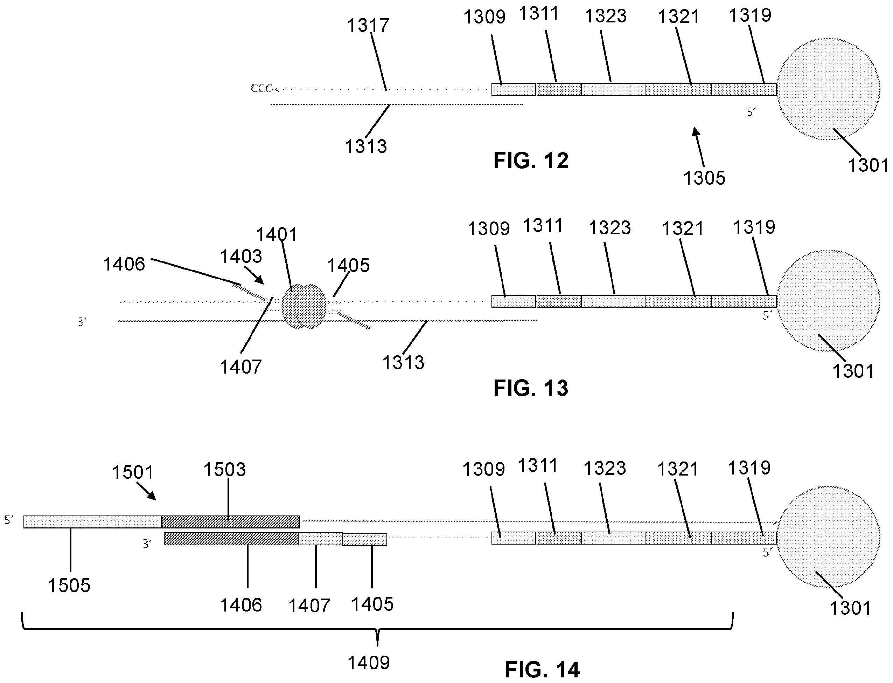

FIGS. 12-15 show exemplary methods for making libraries by direct tagmentation methods with template particles.

FIG. 12 shows a template particle 1301 linked to a capture oligo. As shown, the particle 1301 is linked to (among other things) mRNA capture oligos 1305 that include a 3' poly-T region 1309 (although sequence-specific primers or random N-mers may be used). Where the sample includes cell-free RNA, the capture oligo hybridizes by Watson-Crick base-pairing to a target in the RNA and serves as a primer for reverse transcriptase, which makes a cDNA copy of the RNA. Where the initial sample includes intact cells, the same logic applies but the hybridizing and reverse transcription occurs once a cell releases RNA (e.g., by being lysed).

In preferred embodiments, the target RNAs are mRNAs 1313. Where the target RNAs are mRNAs, the particles 1301 may include mRNA capture oligos 1305 used to at least synthesize a first cDNA copy 1317 of an mRNA 1313. The particles 1301 may further include cDNA capture oligos with 3' portions that hybridize to cDNA copies of the mRNA. For the cDNA capture oligos, the 3' portions may include gene-specific sequences or hexamers. As shown, each of the mRNA capture oligos 1305 may include, from 5' to 3', a SMART site 1319, a PE1 sequence 1321, a cell or droplet barcode 1323, and a poly-T segment 1309. Optionally, the capture oligos may include a UMI 1311.

As shown, the capture oligo 1305 hybridizes to the mRNA 1313. A reverse transcriptase binds and initiates synthesis of a cDNA copy 1317 of the mRNA 1313 to make an RNA/DNA hybrid. Note that the mRNA 1313 is connected to the particle 1301 non-covalently, by Watson-Crick base-pairing. The cDNA 1317 that is synthesized may be covalently linked to the particle 1317 by virtue of the phosphodiester bonds formed by the reverse transcriptase.

FIG. 13 shows a transposase (1401) bound with the RNA/DNA hybrid. The transposase (1401), which is preferably a Tn5 transposase, is attached with adapters 1403 for attaching onto the 5' end of the cDNA 1317. The Tn5 cuts the RNA/DNA hybrids and the adapters 1403 are ligated onto the ends of the cDNA 1317 at random. Preferably the adapter 1403 includes a random N-mer (MDE) 1405, and optionally, a primer (Read 2) 1406 for attaching a sequencing adapter. At this stage, RNaseH may be introduced to degrade the mRNA 1313.

FIG. 14 illustrates the addition and extension of a sequencing adapter 1501 to create a polynucleotide 1409. The adapter 1501 includes a first sequence 1503 complementary to the Read 2 primer 1406 and a sequencing primer 1505, such as P7. The adapter 1501 will hybridize to, and prime the copying of, DNA to create a polynucleotide 1409 with the sequencing adapter. Afterwards, the polynucleotide can be separated from the particle and made into a final library product.

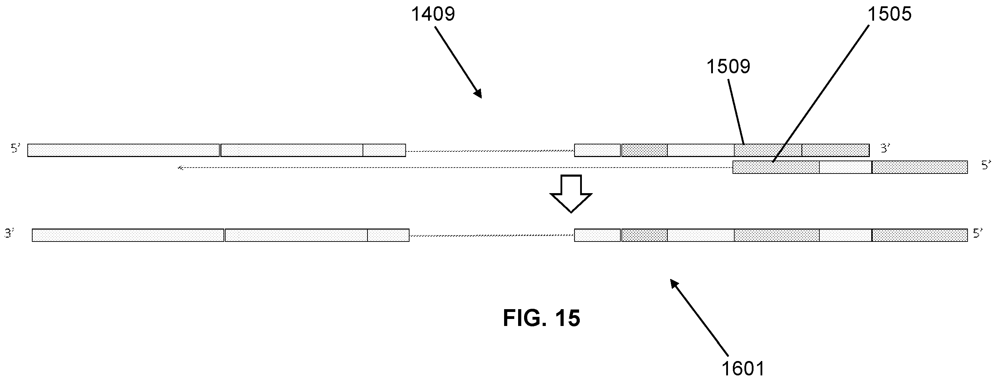

FIG. 15 illustrates the formation of a final library product 1601. In this example, the final library product 1601 is formed by the PCR-based extension a P5-PE1 primer 1505 that is complementary to the PE1 1509 of the released polynucleotide 1409. Extension of the P5-PE1 primer 1505 by PCR creates the final library product 1601. In some embodiments, the P5-PE1 primer 1505 may include indexes, such as an 15 index, and a P5 index. The final library product may be amplified by PCR in advance of sequencing.

Any one of the above described strategies and methods, or combinations thereof may be used in the conjunction particle-templated emulsions. For example, methods may be used for single cell expression profiling, which may include combining target cells with a plurality of template particles in a first fluid to provide a mixture in a reaction tube. The mixture may be incubated to allow association of the plurality of the template particles with target cells. A portion of the plurality of template particles may become associated with the target cells. The mixture is then combined with a second fluid which is immiscible with the first fluid. The fluid and the mixture are then sheared so that a plurality of monodisperse droplets is generated within the reaction tube. The monodisperse droplets generated comprise (i) at least a portion of the mixture, (ii) a single template particle, and (iii) a single target particle. Of note, in practicing methods of the invention provided by this disclosure a substantial number of the monodisperse droplets generated will comprise a single template particle and a single target particle, however, in some instances, a portion of the monodisperse droplets may comprise none or more than one template particle or target cell.

In some aspects, generating the template particles-based monodisperse droplets involves shearing two liquid phases. The mixture is the aqueous phase and, in some embodiments, comprises reagents selected from, for example, buffers, salts, lytic enzymes (e.g. proteinase k) and/or other lytic reagents (e. g. Triton X-100, Tween-20, IGEPAL, bm 135, or combinations thereof), nucleic acid synthesis reagents e.g. nucleic acid amplification reagents or reverse transcription mix, or combinations thereof. The fluid is the continuous phase and may be an immiscible oil such as fluorocarbon oil, a silicone oil, or a hydrocarbon oil, or a combination thereof. In some embodiments, the fluid may comprise reagents such as surfactants (e.g. octylphenol ethoxylate and/or octylphenoxypolyethoxyethanol), reducing agents (e.g. DTT, beta mercaptoethanol, or combinations thereof).

Some methods of the disclosure use oligos. Oligos, sometimes referred to as oligonucleotides, are sequences of contiguous nucleotides of DNA, RNA, or a mixture thereof Preferably, oligos comprise DNA. However, in certain embodiments, oligos may comprise RNA. In other embodiments, oligos may comprise a mixture of DNA and RNA. Oligos may comprise noncanonical nucleotides, such as, synthetic nucleotides that have been modified to incorporate certain biomolecular properties. The length of the oligo is usually denoted by "-mer". For example, an oligo of six nucleotides is a hexamer, or 6-mer, while one of 25 nucleotides may be referred to as a 25-mer.

Some aspects of the invention rely on oligos comprising random sequences of contiguous nucleotides, i.e., random N-mers. A random N-mer is an oligo, or a portion of an oligo, that includes a random or quasi random sequence of nucleotides, the length of which is denoted by "N". For example, a random 5-mer is an oligo of five contiguous nucleotides. According to aspects of the invention, random N-mers, for example, a random 5-mer, are useful for enhancing molecular diversity within a population of nucleic acids. The is because adding random N-mers to the ends of a population of identical nucleic acids generates distinguishable nucleic acids on account of the different N-mers. Accordingly, in some instances, random N-mers may be referred to as molecular diversity enhancers (MDEs).

## Claims

1. A method for preparing a sequencing library, the method comprising: obtaining a sample comprising RNA; fragmenting the RNA to produce fragments with cleaved ends at random cleavage locations; adding oligos at the cleaved ends; and reverse transcribing the fragments and oligos to make polynucleotides with unique labels, wherein the unique labels are defined by different combinations of the random cleavage locations and the oligos.

2. The method of claim 1, wherein the oligos comprise random N-mers.

3. The method of claim 2, wherein the random N-mers provide 4^N different sequence combinations and wherein a total number of the sequence combinations provided by the random N-mers is substantially less than an amount of distinct species of RNA present in the sample.

4. The method of claim 1, wherein fragmenting comprises metal-ion catalysis of RNA.

5. The method of claim 1, wherein the sample further comprises a mixture with cells, and wherein the method further comprises partitioning the mixture into droplets that each include one or zero cells and lysing the cells within the droplets to release the RNA.

6. The method of claim 5, wherein the mixture comprises a plurality of particles that template the formation of the droplets.

7. The method of claim 6, wherein fragmenting occurs within the droplets coincident with cell lysis.

8. The method of claim 6, wherein the particles comprise reagents for cell lysis, RNA fragmentation, or reverse transcription.

9. The method of claim 6, wherein the particles comprise capture poly-T sequences that hybridize to poly-A tails of a portion of the fragments.

10. The method of claim 9, wherein, after hybridization, the fragments are reverse transcribed into complementary DNA.

11. The method of claim 1, wherein the oligos comprise template switching oligos and random N-mers.

12. The method of claim 11, wherein reverse transcribing the fragments comprises reverse transcriptase enzymes that add additional nucleotides to ends of the cDNA after reaching the cleaved ends of the fragments, wherein the additional nucleotides provide overhangs.

13. The method of claim 12, wherein template switching oligos attach to the overhangs and provide additional template that is copied into the cDNA to thereby create polynucleotides comprising the random N-mers and the random cleavage locations.

14. The method of claim 1, further comprising amplifying the polynucleotides to create amplicons, and sequencing the amplicons to create a plurality of sequence reads.

15. The method of claim 14, further comprising analyzing the sequence reads to identify PCR duplicates, wherein analyzing comprises aligning the sequence reads to a reference genome and determining genomic coordinates that correspond with the random cleavage locations.

## Patentansprüche

1. Verfahren zum Herstellen einer Sequenzierungsbibliothek, wobei das Verfahren umfasst: Erhalten einer Probe, die RNA umfasst; Fragmentieren der RNA, um Fragmente mit gespaltenen Enden an zufälligen Spaltstellen zu erzeugen; Hinzufügen von Oligos an den gespaltenen Enden; und reverses Transkribieren der Fragmente und Oligos, um Polynukleotide mit eindeutigen Markierungen herzustellen, wobei die eindeutigen Markierungen durch verschiedene Kombinationen der zufälligen Spaltstellen und der Oligos definiert sind.

2. Verfahren nach Anspruch 1, wobei die Oligos zufällige N-Mere umfassen.

3. Verfahren nach Anspruch 2, wobei die zufälligen N-Mere 4^N verschiedene Sequenzkombinationen liefern und wobei die Gesamtzahl der von den zufälligen N-Meren gelieferten Sequenzkombinationen im Wesentlichen geringer ist als die Menge der in der Probe vorhandenen unterschiedlichen RNA-Spezies.

4. Verfahren nach Anspruch 1, wobei das Fragmentieren Metallionen-Katalyse von RNA umfasst.

5. Verfahren nach Anspruch 1, wobei die Probe ferner ein Gemisch mit Zellen umfasst, und wobei das Verfahren ferner das Aufteilen des Gemischs in Tröpfchen, die jeweils eine oder null Zellen enthalten, und das Lysieren der Zellen in den Tröpfchen umfasst, um die RNA freizusetzen.

6. Verfahren nach Anspruch 5, wobei das Gemisch eine Vielzahl von Partikeln umfasst, die die Bildung der Tröpfchen vorgeben.

7. Verfahren nach Anspruch 6, wobei das Fragmentieren in den Tröpfchen zeitgleich mit der Zelllyse erfolgt.

8. Verfahren nach Anspruch 6, wobei die Partikel Reagenzien für Zelllyse, RNA-Fragmentierung oder reverse Transkription umfassen.

9. Verfahren nach Anspruch 6, wobei die Partikel Capture-Poly-T-Einfangsequenzen umfassen, die an Poly-A-Schwänze eines Teils der Fragmente hybridisieren.

10. Verfahren nach Anspruch 9, wobei die Fragmente nach der Hybridisierung in komplementäre DNA revers transkribiert werden.

11. Verfahren nach Anspruch 1, wobei die Oligos Template-Switching-Oligos und zufällige N-Mere umfassen.

12. Verfahren nach Anspruch 11, wobei das reverse Transkribieren der Fragmente reverse Transkriptaseenzyme umfasst, die zusätzliche Nukleotide an Enden der cDNA anfügen, nachdem sie die gespaltenen Enden der Fragmente erreicht haben, wobei die zusätzlichen Nukleotide Überhänge bereitstellen.

13. Verfahren nach Anspruch 12, wobei Template-Switching-Oligos an die Überhänge binden und ein zusätzliches Template bereitstellen, das in die cDNA kopiert wird, um dadurch Polynukleotide zu erzeugen, die die zufälligen N-Mere und die zufälligen Spaltstellen umfassen.

14. Verfahren nach Anspruch 1, ferner umfassend das Amplifizieren der Polynukleotide zur Erzeugung von Amplicons und das Sequenzieren der Amplicons zur Erzeugung einer Vielzahl von Sequenzlesungen.

15. Verfahren nach Anspruch 14, ferner umfassend das Analysieren der Sequenzlesungen, um PCR-Duplikate zu identifizieren, wobei das Analysieren das Ausrichten der Sequenzlesungen an einem Referenzgenom und das Bestimmen genomischer Koordinaten umfasst, die mit den zufälligen Spaltstellen übereinstimmen.

## Revendications

1. Procédé de préparation d'une banque de séquençage, le procédé comprenant : l'obtention d'un échantillon comprenant de l'ARN ; la fragmentation de l'ARN pour produire des fragments avec des extrémités clivées à des emplacements de clivage aléatoires ; l'ajout d'oligos aux extrémités clivées ; et la transcription inverse des fragments et des oligos pour fabriquer des polynucléotides avec des étiquettes uniques, dans lequel les étiquettes uniques sont définies par différentes combinaisons des emplacements de clivage aléatoires et des oligos.

2. Procédé selon la revendication 1, dans lequel les oligos comprennent des N-mères aléatoires.

3. Procédé selon la revendication 2, dans lequel les N-mères aléatoires fournissent 4^N combinaisons de séquences différentes et dans lequel un nombre total de combinaisons de séquences fournies par les N-mères aléatoires est sensiblement inférieur à une quantité d'espèces distinctes d'ARN présentes dans l'échantillon.

4. Procédé selon la revendication 1, dans lequel la fragmentation comprend une catalyse par ions métalliques de l'ARN.

5. Procédé selon la revendication 1, dans lequel l'échantillon comprend également un mélange avec des cellules, et dans lequel le procédé comprend également la partition du mélange en gouttelettes qui comportent chacune une ou zéro cellule et la lyse des cellules dans les gouttelettes pour libérer l'ARN.

6. Procédé selon la revendication 5, dans lequel le mélange comprend une pluralité de particules qui modèlent la formation des gouttelettes.

7. Procédé selon la revendication 6, dans lequel la fragmentation se produit dans les gouttelettes en même temps que la lyse cellulaire.

8. Procédé selon la revendication 6, dans lequel les particules comprennent des réactifs pour la lyse cellulaire, la fragmentation de l'ARN, ou la transcription inverse.

9. Procédé selon la revendication 6, dans lequel les particules comprennent des séquences poly-T de capture qui s'hybrident aux queues poly-A d'une partie des fragments.

10. Procédé selon la revendication 9, dans lequel, après hybridation, les fragments sont rétrotranscrits en ADN complémentaire.

11. Procédé selon la revendication 1, dans lequel les oligos comprennent des oligos à commutation de modèle et des N-mères aléatoires.

12. Procédé selon la revendication 11, dans lequel la transcription inverse des fragments comprend des enzymes de transcriptase inverse qui ajoutent des nucléotides supplémentaires aux extrémités de l'ADNc après avoir atteint les extrémités clivées des fragments, dans lequel les nucléotides supplémentaires fournissent des surplombs.

13. Procédé selon la revendication 12, dans lequel les oligos de commutation de modèle se fixent aux surplombs et fournissent un modèle supplémentaire qui est copié dans l'ADNc pour ainsi créer des polynucléotides comprenant les N-mères aléatoires et les emplacements de clivage aléatoires.

14. Procédé selon la revendication 1, comprenant également l'amplification des polynucléotides pour créer des amplicons, et le séquençage des amplicons pour créer une pluralité de lectures de séquences.

15. Procédé selon la revendication 14, comprenant également l'analyse des lectures de séquence pour identifier les doublons de PCR, dans lequel l'analyse comprend l'alignement des lectures de séquence sur un génome de référence et la détermination des coordonnées génomiques qui correspondent aux emplacements de clivage aléatoire.
